# FASCICULE DE BREVET EUROPEEN

(11) **EP 2 295 012 B1**
(45) Date de publication et mention de la délivrance du brevet: **09.05.2012**
(21) Numéro de dépôt: 10175690.6
(22) Date de dépôt: 08.09.2010
(51) Int. Cl.: A61F 13/15, A41B 9/00, A41B 9/12

(54) **Article vestimentaire de la famille des culottes à barrières d'étanchéité**
Kleidungsstück in Slip-Form mit Feuchtigkeitsperre
Panty type garment with moisture barrier

(30) Priorité: 09.09.2009 FR 0904327
(43) Date de publication de la demande: 16.03.2011
(73) Titulaire: PIWAPEE, 79410 Cherveux (FR)
(72) Inventeur: Tirmarche, Estelle, 60500, CHANTILLY (FR)
(74) Mandataire: Godineau, Valérie

(56) Documents cités:
- EP-A1- 1 600 067
- EP-A1- 1 836 910
- EP-A2- 1 166 738
- WO-A1-98/37842
- WO-A1-2008/008743
- US-A1- 2005 145 150

## Description

La présente invention concerne de manière générale les articles vestimentaires de la famille des culottes tels que, couche, couche-culotte, surculotte, culotte de bain ou maillot-couche, slip.

L'invention concerne plus particulièrement un article vestimentaire de la famille des culottes comportant un corps de culotte principal comprenant une portion avant et une portion arrière de corps de culotte reliées entre elles par une portion d'entrejambes et raccordées ou raccordables l'une à l'autre pour former, à l'état raccordé, une ouverture de ceinture et deux ouvertures de jambe. Ladite portion d'entrejambes est équipée d'au moins une aile, appelée aile d'étanchéité, formant barrière d'étanchéité du côté de chaque ouverture de jambe.

On connaît de l'état de la technique, et notamment du document WO 98/37842, des articles tels que décrits ci-dessus. Pour ces articles, les barrières d'étanchéité se présentent sous forme de goussets constitués chacun d'une pièce en forme de croissant de lune rapportée sur un bord d'ouverture de jambe au niveau de la portion d'entrejambe. Ainsi, les deux goussets sont formés de deux pièces distinctes de la partie du fond de culotte destinée à venir en contact de l'enfant, ce qui complique la conception de l'article. En particulier, la liaison entre chaque gousset en croissant de lune et la partie de fond de culotte au niveau d'un bord de la portion d'entrejambes est longue et compliquée à réaliser. En outre, les performances d'étanchéité obtenues avec de tels goussets, formés de pièces en demi-lune rapportées sur les bords d'ouverture de jambe, ne sont pas satisfaisantes.

La présente invention a pour but de proposer un article vestimentaire de la famille des culottes pour lequel le risque de fuite est réduit.

Un autre but de la présente invention est de proposer un article vestimentaire de la famille des culottes dont les barrières d'étanchéité au niveau de l'entrejambes sont formées avec un nombre réduit de pièce, de manière simple et rapide.

A cet effet, l'invention a pour objet un article vestimentaire selon la revendication 1 de la famille des culottes, tel que couche, couche-culotte, surculotte, culotte de bain ou maillot-couche, slip, ledit article comportant un corps de culotte principal comprenant une portion avant et une portion arrière de corps de culotte reliées entre elles par une portion d'entrejambes et raccordées ou raccordables l'une à l'autre pour former, à l'état raccordé, une ouverture de ceinture et deux ouvertures de jambe, ladite portion d'entrejambes étant équipée d'au moins une aile, appelée aile d'étanchéité, formant barrière d'étanchéité du côté de chaque ouverture de jambe,
caractérisé en ce que ledit article comprend en outre une bande dite de propreté disposée côté intérieur du corps de culotte principal, cette bande de propreté s'étendant longitudinalement entre les bords d'ouverture de ceinture de la portion avant et de la portion arrière du corps principal en venant à recouvrement au moins partiel de la portion d'entrejambes dudit corps principal,
et en ce que la bande de propreté est solidarisée au corps de culotte principal par au moins deux lignes de couture dites longitudinales s'étendant le long des bords longitudinaux de ladite bande, chaque ligne de couture étant écartée du bord longitudinal de la bande le plus proche de ladite ligne de couture pour délimiter, entre ladite ligne de couture et ledit bord longitudinal, l'une desdites ailes d'étanchéité de sorte chacune desdites ailes d'étanchéité est formée d'une seule pièce avec ladite bande de propreté.

La bande de propreté est encore appelée hamac, du fait de la forme générale concave que confèrent les ailes d'étanchéité à la bande de propreté. Les ailes délimitées entre les bords longitudinaux du hamac et les coutures longitudinales de solidarisation du hamac au corps de culotte permettent d'empêcher les fuites au niveau de l'entrejambes de l'enfant qui est équipé d'une telle couche et/ou culotte.

La couture des parties longitudinales du hamac sur le corps de culotte principal, par exemple sur la partie de fond de la culotte ou sur une partie solidaire de ladite partie de fond de la culotte, permet de former entre chaque bord libre longitudinal du hamac et la ligne de couture correspondante, une aile qui peut se redresser par rapport à la partie centrale du hamac délimitée entre les deux lignes de couture. Les ailes forment ainsi des barrières anti-fuite efficaces. En effet, chaque ligne de couture qui s'étend sensiblement parallèlement à un bord longitudinal du hamac forme une ligne de pliage autour de laquelle ledit bord longitudinal correspondant du hamac peut être relevé.

Autrement dit, le fait de pratiquer deux lignes de couture longitudinales sensiblement parallèles entre ledit hamac et le corps de culotte principal, à écartement l'une de l'autre et des bords longitudinaux, permet de délimiter deux ailes et de définir entre les deux lignes de couture une partie centrale du hamac qui est solidarisée par les deux lignes de couture au fond de culotte, tandis que les ailes, définies entre les bords longitudinaux libres et les coutures, peuvent se redresser autour des zones de pliage définies par les coutures. Les ailes peuvent ainsi se relever par rapport au fond de culotte pour venir contre les jambes de l'enfant et former des barrières anti-fuites.

Pour former les deux ailes de l'article selon l'invention, il suffit, contrairement aux solutions connues de l'état de la technique, de rapporter une seule pièce, à savoir la bande de propreté, ou hamac, sur le corps de culotte principal et de réaliser simplement deux coutures le long et à écartement des bords longitudinaux du hamac pour solidariser le hamac au corps de culotte principal. En outre, le hamac, dont les bords longitudinaux servent à former les ailes anti-fuites, permet de contenir l'humidité de l'article et évite que l'humidité se répande sur les cuisses de l'enfant.

Enfin, contrairement à la solution connue utilisant des goussets formés de pièces en demi-lunes qui génèrent une surépaisseur de l'article au niveau de l'entrejambe, les ailes de l'article selon l'invention formées dans la continuité de la partie centrale du hamac permettent de limiter l'épaisseur de l'article, en particulier au niveau de l'entrejambe. En outre, de telles ailes anti-fuite formées d'un seul tenant avec la partie centrale du hamac permettent d'augmenter l'échancrure des ouvertures de jambe, ce qui facilite les mouvements de l'enfant.

Selon l'invention, chacune desdites ailes d'étanchéité formées d'une pièce avec ladite bande de propreté est équipée sur au moins une partie de sa longueur de moyens de raccourcissement de la longueur de son bord longitudinal libre permettant de relever ladite aile d'étanchéité par rapport à la portion de la bande de propreté qui s'étend entre lesdites ailes.

Selon l'invention, lesdits moyens de raccourcissement de chaque aile d'étanchéité comprennent un élastique de mise en tension du bord longitudinal libre de l'aile, qui s'étend sur au moins une partie de la longueur de ladite aile d'étanchéité.

L'élastique permet de raccourcir la longueur du bord longitudinal libre de l'aile et ainsi de redresser ladite aile correspondante par rapport au fond du hamac.

La combinaison des coutures longitudinales du hamac sur le corps de culotte et des élastiques au niveau des ailes ainsi formées, permet aux ailes de venir épouser l'intérieur de la cuisse de l'enfant au niveau de l'entrejambes en se redressant par rapport au fond de culotte, ce qui assure la formation d'une barrière anti-fuite efficace au niveau de la zone d'entrejambes de l'enfant.

Avantageusement, le bord longitudinal de la bande est libre au moins au niveau de la portion d'entrejambes pour permettre à ladite aile de se relever par rapport au corps de culotte principal afin de former une barrière d'étanchéité en s'adaptant par son bord libre au profil de la jambe à entourer.

Selon une caractéristique avantageuse de l'invention, chaque élastique s'étend sur une partie seulement de la longueur de ladite aile, de préférence sur la longueur de la portion d'entrejambe, en étant écarté des extrémités de ladite aile.

Chaque élastique est écarté des bords de ceinture des portions avant et arrière du corps de culotte principal, ce qui permet à l'état défait de la couche qu'elle ne se mette pas en boule. En outre, l'absence d'élastique à proximité des bords de ceinture des portions avant et arrière du corps de culotte principal permet à l'article de s'adapter à différentes morphologies tout en se resserrant au niveau de l'entrejambes de l'enfant pour éviter des fuites.

Selon une caractéristique avantageuse de l'invention, les extrémités du bord longitudinal délimitant chaque aile sont solidarisées, de préférence par couture, au corps de culotte principal, de préférence respectivement aux, ou au voisinage des, bords d'ouverture de ceinture de la portion avant et de la portion arrière du corps principal.

Selon une caractéristique avantageuse de l'invention, le corps de culotte principal est un multicouches formé d'au moins une couche externe imperméable et d'une couche interne. Selon une variante de réalisation, on peut prévoir que la couche externe soit perméable.

Selon une caractéristique avantageuse de l'invention, la couche interne du corps de culotte principal est en matériau perméable, tel que de la fibre de coton.

Selon une caractéristique avantageuse de l'invention, au moins les portions de la bande de propreté servant à former les ailes sont imperméables, côté intérieur et/ou extérieur.

Selon une caractéristique avantageuse de l'invention, la bande de propreté comprend, entre les deux coutures de solidarisation au corps de culotte principal, des moyens de fixation d'un élément absorbant équipé de moyens de fixation complémentaires pour une fixation amovible.

Selon une caractéristique avantageuse de l'invention, ladite bande de propreté comprend un élément d'absorption situé entre les deux lignes de coutures longitudinales.

L'invention sera bien comprise à la lecture de la description suivante d'exemples de réalisation, en référence aux dessins annexés dans lesquels :
- la figure 1 est une vue de dessus du corps de culotte principal déployé et du hamac avant assemblage par couture ;
- la figure 2 est une vue du corps de culotte principal et du hamac de la figure 1 à l'état assemblé par couture de manière à former les ailes à fonction de barrière d'étanchéité ;

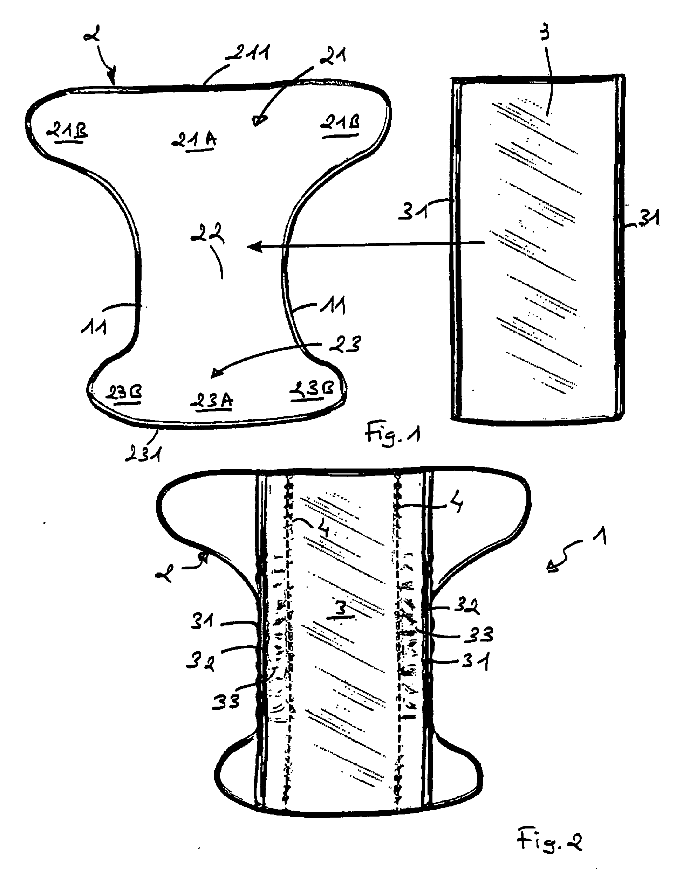

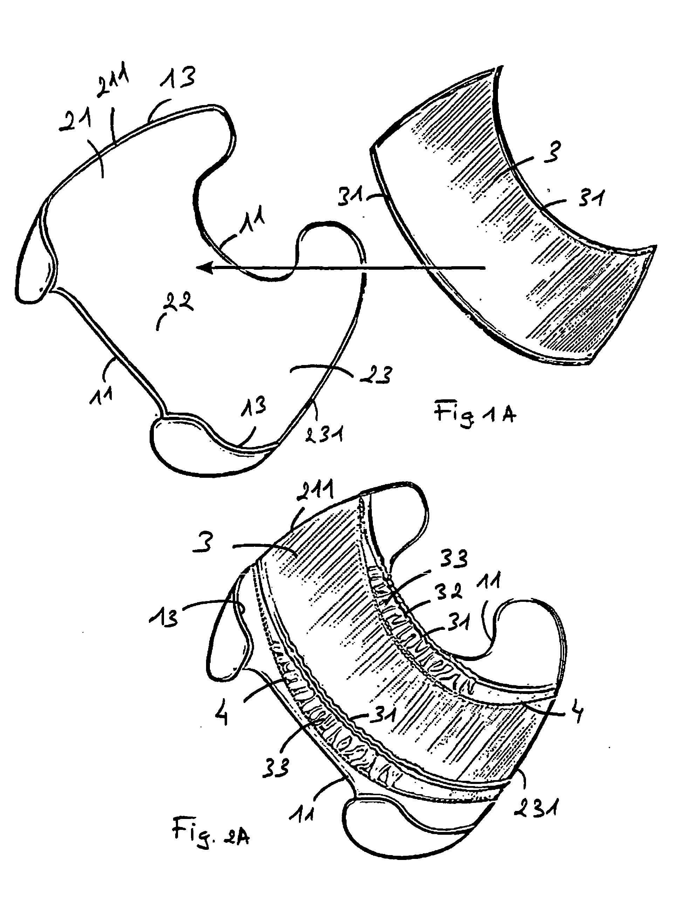

- les figures 1A et 2A sont des vues en perspective respectivement des figures 1 et 2 ;

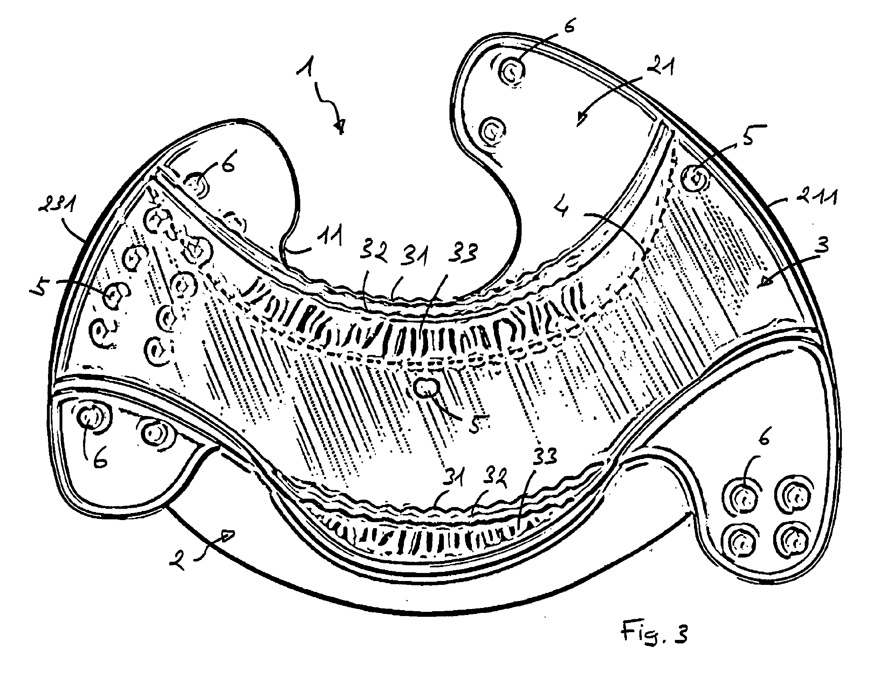

- la figure 3 est une vue d'une couche-culotte selon l'invention formée par assemblage par couture d'un corps principal et d'un hamac sur lequel peut être fixé un élément absorbant.

Il doit être noté qu'on entend par "culotte" un article pour lequel les portions avant et arrière qui délimitent l'ouverture de ceinture sont détachables ou non l'une par rapport à l'autre.

En référence aux figures et comme rappelé ci-dessus, l'invention concerne un article vestimentaire 1, de préférence lavable, de la famille des culottes tel que slip, couche-culotte, surculotte, culotte de bain ou maillot-couche.

Ledit article comporte un corps de culotte 2 principal comprenant une portion avant 23, correspondant à la portion de l'article destinée à venir contre le bas du ventre de l'enfant, et une portion arrière 21 de corps de culotte, correspondant à la portion de l'article destinée à venir contre le bas du dos de l'enfant. Les portions avant 23 et arrière 21 sont reliées entre elles par une portion d'entrejambes 22. Les portions avant 23 et arrière 21 sont raccordables ou raccordées l'une à l'autre pour former une ouverture de ceinture 13 et deux ouvertures de jambe 11. Chaque ouverture 11, 13 est entourée sur au moins une partie de son pourtour d'un élastique permettant à la portion d'entrejambes de s'adapter à la morphologie de l'enfant au niveau de la taille et de l'entrejambe.

Dans l'exemple illustré aux figures, la portion d'entrejambes 22 forme un étranglement par rapport aux portions avant et arrière du corps principal. Autrement dit, chaque portion avant 23 et arrière 21 présente, d'une part, une zone centrale 23A, 21A située dans le prolongement de la portion d'entrejambes et, d'autre part, des zones d'extrémité latérales formant des oreilles 23B, 21 B qui sont raccordables aux oreilles 21 B, 23B de l'autre portion.

Dans l'exemple illustré aux figures, les portions avant 23 et arrière 21 sont raccordables l'une à l'autre à l'aide de moyens de fixation complémentaires 6, tels que des boutons-pressions. Avantageusement, au moins l'une des portions avant et arrière comporte une pluralité de moyens de fixation complémentaires écartés les uns des autres de manière à permettre un réglage de la dimension d'ouverture de ceinture et/ou de la dimension d'ouverture d'entrejambes en fonction de la taille et de la morphologie de l'enfant. En variante, les portions avant 23 et arrière 21 peuvent être raccordées de manière permanente à la manière d'un slip.

L'article 1 comporte en outre une bande 3, dite de propreté, disposée côté intérieur du corps de culotte principal, c'est-à-dire du côté du corps de culotte orienté vers l'enfant à l'état équipé dudit article. Plus précisément, ladite bande de propreté est destinée à se trouver en coïncidence avec l'anus et le méat urinaire de l'utilisateur. Cette bande de propreté vient doubler intérieurement le corps de culotte principal. Cette bande 3, encore appelée "hamac", s'étend longitudinalement entre les bords d'ouverture 231, 211 de ceinture de la portion avant 23 et de la portion arrière 21 du corps principal en venant à recouvrement au moins partiel de la portion d'entrejambes 22 dudit corps principal. Ainsi dans l'exemple illustré plus particulièrement à la figure 2, la bande recouvre les zones centrales 23A, 21A des portions avant et arrière ainsi que la portion d'entrejambes 22. En particulier, le hamac est une bande sensiblement rectangulaire, de préférence de largeur sensiblement égale à la largeur de la portion d'entrejambes. Ainsi, la bande de propreté s'étend côté intérieur de la culotte entre les oreilles des portions avant et arrière. Les bords longitudinaux de la bande s'étendent sensiblement parallèlement aux bords latéraux de la portion d'entrejambe.

La bande 3 de propreté, ou hamac, est solidarisée au corps de culotte 2 principal par au moins deux lignes de couture 4 dites longitudinales s'étendant le long et à écartement des bords longitudinaux 31 de ladite bande. Les lignes de couture 4 sont écartées des bords 31 de la bande pour ménager entre chaque ligne de couture 4 et le bord longitudinal 31 le plus proche de ladite ligne, une aile 33 dite aile d'étanchéité.

Chaque aile présente une largeur comprise avantageusement dans la plage [2 cm - 10 cm] et, de préférence, dans la plage [3 cm - 5 cm].

Le bord longitudinal 31 de la bande 3 est libre au moins au niveau de la portion d'entrejambes pour permettre à ladite aile de se relever par rapport au fond de culotte formé par le corps de culotte principal, en particulier par rapport à la portion d'entrejambes 22, afin de former une barrière d'étanchéité en s'adaptant par son bord libre au profil de l'intérieur de la cuisse de l'enfant.

Chaque aile 33 est équipée sur au moins une partie de sa longueur de moyens de raccourcissement 32 de la longueur de son bord libre 31, afin de maintenir relevée l'aile par rapport au fond de culotte. De préférence, les moyens de raccourcissement sont présents au moins sur la longueur d'aile s'étendant le long de la portion d'entrejambe. A cet effet, dans l'exemple illustré aux figures, chaque aile 33 est équipée d'un élastique 32 qui s'étend sur une longueur correspondant à la longueur de la portion d'entrejambe 22. Avantageusement, un élément formant gaine est fixé, par exemple par couture, autour du bord longitudinal 31 de l'aile 33 où un ourlet est réalisé de manière à envelopper l'élastique 32. L'élastique 32 est étiré puis fixé à l'état étiré au bord longitudinal 31 libre de l'aile 33. Cet élastique est relâché après la fixation pour produire des plis, plissés ou froncés, aux fins de permettre un relevé de l'aile et une adaptation ultérieure du bord libre de profil de la jambe à entourer.

Préférentiellement, chaque élastique est écarté des extrémités de l'aile 33 correspondante, c'est-à-dire des extrémités des bords longitudinaux 31.

La présence de l'élastique sur la longueur de l'aile correspondant à la longueur de la portion d'entrejambes permet d'assurer un bon redressement de l'aile par rapport à face de fond de culotte et ainsi la formation d'une barrière d'étanchéité fiable au niveau de l'entrejambe. En outre, lorsque les parties de l'aile qui s'étendent au niveau des portions avant et arrière du corps de culotte principal sont dépourvues d'élastique, l'article, à l'état détaché entre elles de ses parties avant et arrière, ne se met pas en boule sur lui-même, ce qui facilite sa manipulation, en particulier pour habiller l'enfant.

Le bord longitudinal 31 délimitant chaque aile 33, en coopération avec la couture longitudinale 4 correspondante, est solidarisé d'une part à la portion avant 23 et, d'autre part, à la portion arrière 21 du corps de culotte 2 principal en laissant libre au moins la longueur du bord longitudinal 31 située au niveau de l'entrejambes pour permettre le redressement de l'aile au niveau de l'entrejambes. Dans l'exemple illustré aux figures, les extrémités de chaque bord longitudinal de la bande sont solidarisées, par exemple par couture, respectivement aux bords d'ouverture de ceinture 211, 231 des portions avant et arrière du corps de culotte 2 principal. Les lignes de couture longitudinales s'étendent entre les bords d'ouverture de ceinture des portions avant et arrière.

La solidarisation de chaque bord longitudinal de la bande aux portions avant et arrière du corps de culotte peut s'effectuer directement ou indirectement par l'intermédiaire d'une pièce ou couche de textile elle-même solidaire du corps principal de culotte.

De préférence, au moins les portions de la bande de propreté servant à former les ailes sont imperméables, côté intérieur et/ou extérieur. A cet effet, lesdites ailes sont réalisées en matériau imperméable et/ou traitées pour être imperméables côté intérieur et/ou extérieur.

Dans l'exemple illustré aux figures, le corps de culotte principal est un multicouches formé d'au moins une couche externe imperméable et d'une couche interne. La couture de la bande 3 au corps de culotte 2 principal s'effectue plus précisément entre la bande 3 et la couche interne (c'est-à-dire la couche orientée côté enfant) pour ne pas faire apparaître la couture sur la couche externe et ainsi assurer l'imperméabilité de ladite couche externe.

La couche externe ou face extérieure du corps de culotte principal est étanche pour limiter les fuites par capillarité vers les autres vêtements ou pour éviter que l'article ne se remplisse d'eau dans le cas d'un maillot-couche.

En outre, dans le cas où l'article de vêtement selon l'invention forme un maillot-couche destiné à aller dans l'eau, les ailes forment une barrière d'étanchéité en particulier de l'extérieur vers l'intérieur, ce qui permet à l'article de ne pas se charger en eau et ainsi de rester léger pour l'enfant. Bien entendu les ailes forment également barrière d'étanchéité de l'intérieur vers l'extérieur de l'article, ce qui permet d'éviter des fuites, en particulier lorsque l'enfant est hors d'eau.

On peut prévoir que la couche interne du corps de culotte 2 principal soit en matériau perméable, tel que de la fibre de coton.

Comme illustré à la figure 3, on peut prévoir que la bande 3 de propreté comprenne entre les deux coutures 4 de solidarisation au corps de culotte 2 principal des moyens de fixation 5 d'un élément absorbant.

On peut également prévoir que ladite bande de propreté 3 comprenne un élément absorbant. Ledit élément absorbant est de préférence situé entre les deux coutures 4 pour laisser la possibilité aux ailes 33 de se redresser librement autour des coutures afin de former les barrières d'étanchéité. Ledit élément absorbant peut être intégré à l'intérieur de la bande dans le cas d'une bande multicouche ou être situé du côté de la face de la bande destinée à venir en contact avec la peau de l'enfant.

Dans le cas d'une couche-culotte, la face intérieure du corps principal de la couche est absorbante. Le hamac est alors cousu sur la face absorbante du corps principal de la couche.

Comme rappelé ci-dessus, l'article selon l'invention peut également être une surculotte qui vient s'ajuster sur une couche lavable afin de limiter les risques de fuite pendant la nuit. Dans ce cas, avantageusement, le hamac est en matériau imperméable. De même, le côté intérieur du corps de culotte principal peut être imperméable. On prévoit également que la surculotte soit équipée de goussets supplémentaires, formés de manière classique à l'aide de deux pièces distinctes en demi-lune, qui sont rapportées chacune par couture sur un bord de la portion d'entrejambes qui définit le bord d'ouverture d'une jambe. Ainsi, la surculotte comprend pour chaque jambe deux niveaux de goussets, l'un formé par une aile du hamac et l'autre formé par la pièce en demi-lune rapportée sur le bord correspondant de la portion d'entrejambe, ce qui permet de réduire les risques de fuite pendant la nuit.

De manière générale, les parties imperméables de l'article peuvent être réalisées en nylon ou en polyester. Les parties absorbantes ou perméables de l'article peuvent être réalisées en coton, bambou, chanvre, polyester de type éponge ou microfibres, et autres fibres naturelles, synthétiques et artificielles absorbantes.

On pourrait envisager de superposer plusieurs hamacs de largeurs différentes qui seraient solidarisés directement ou indirectement au corps de culotte principal de la couche de manière à définir plusieurs niveaux de barrière d'étanchéité. A cet effet on peut prévoir de coudre l'empilement de hamacs sur le corps de culotte en réalisant au moins deux coutures longitudinales telles que décrites ci-dessus. On peut également prévoir de coudre chaque hamac supérieur sur le hamac inférieur correspondant par au moins deux coutures longitudinales telles que décrites ci-dessus.

## Revendications

1. Article vestimentaire (1) de la famille des culottes, tel que couche, couche-culotte, surculotte, culotte de bain ou maillot-couche, slip, ledit article comportant un corps de culotte (2) principal comprenant une portion avant (23) et une portion arrière (21) de corps de culotte reliées entre elles par une portion d'entrejambes (22) et raccordées ou raccordables l'une à l'autre pour former, à l'état raccordé, une ouverture de ceinture (13) et deux ouvertures de jambe (11), ladite portion d'entrejambes étant équipée d'au moins une aile, appelée aile d'étanchéité (33), formant barrière d'étanchéité du côté de chaque ouverture de jambe,
**caractérisé en ce que** ledit article comporte une bande dite de propreté disposée côté intérieur du corps de culotte principal, cette bande (3) s'étendant longitudinalement entre les bords d'ouverture (231, 211) de ceinture de la portion avant (23) et de la portion arrière (21) du corps principal en venant à recouvrement au moins partiel de la portion d'entrejambes (22) dudit corps principal,
et **en ce que** la bande (3) de propreté est solidarisée au corps de culotte (2) principal par au moins deux lignes de couture (4) dites longitudinales s'étendant le long des bords longitudinaux (31) de ladite bande, chaque ligne de couture (4) étant écartée du bord longitudinal (31) de la bande le plus proche de ladite ligne de couture pour délimiter, entre ladite ligne de couture et ledit bord longitudinal, l'une desdites ailes d'étanchéité (33) de sorte que chacune desdites ailes d'étanchéité (33) est formée d'une seule pièce avec ladite bande de propreté (3),
et **en ce que** chacune desdites ailes d'étanchéité (33) formées d'une pièce avec ladite bande de propreté (3) est équipée, sur au moins une partie de sa longueur, d' un élastique (32) de mise en tension du bord longitudinal libre de l'aile qui permet, en raccourcissant la longueur dudit bord longitudinal libre (31) de l'aile, de relever ladite aile d'étanchéité par rapport à la portion de la bande de propreté qui s'étend entre lesdites ailes (33).

2. Article vestimentaire (1) selon la revendication 1, **caractérisé en ce que** chaque élastique s'étend sur une partie seulement de la longueur de ladite aile, de préférence sur la longueur de la portion d'entrejambe, en étant écartés des extrémités de ladite aile.

3. Article vestimentaire (1) selon l'une des revendications précédentes, **caractérisé en ce que** les extrémités du bord longitudinal (31) délimitant chaque aile sont solidarisées, de préférence par couture, au corps de culotte principal, de préférence respectivement aux, ou au voisinage des, bords d'ouverture (231, 211) de ceinture de la portion avant (23) et de la portion arrière (21) du corps principal.

4. Article vestimentaire (1) selon l'une des revendications précédentes, **caractérisé en ce que** le corps de culotte principal est un multicouches formé d'au moins une couche externe imperméable et d'une couche interne.

5. Article vestimentaire (1) selon la revendication précédente, **caractérisé en ce que** la couche interne du corps de culotte (2) principal est en matériau perméable, tel que de la fibre de coton.

6. Article vestimentaire (1) selon l'une des revendications précédentes, **caractérisé en ce qu'**au moins les portions de la bande (3) de propreté servant à former les ailes sont imperméables, côté intérieur et/ou extérieur.

7. Article vestimentaire (1) selon l'une des revendications précédentes, **caractérisé en ce que** la bande de propreté comprend, entre les deux coutures (4) de solidarisation au corps de culotte (2) principal, des moyens de fixation (5) d'un élément absorbant équipé de moyens de fixation complémentaires pour une fixation amovible.

8. Article vestimentaire (1) selon l'une des revendications précédentes, **caractérisé en ce que** ladite bande de propreté comprend un élément d'absorption situé entre les deux lignes de coutures longitudinales.

## Claims

1. A panty-type garment (1), such as a diaper, disposable diaper, trunk, bathing trunks or swimsuit diaper, briefs, said garment comprising a main panty bottom (2) comprising a front portion (23) and a back portion (21) of the panty body connected together by a crotch portion (22) and connected or connectable to one another to form, in the connected state, a waist opening (13) and two leg openings (11), said crotch portion being equipped with at least one wing, called sealing wing (33), forming a moisture barrier on the side of each leg opening,
**characterized in that** said garment comprises a so-called sanitary strip positioned inside the main panty body, said strip (3) extending longitudinally between the waist opening edges (231, 211) of the front portion (23) and the rear portion (21) of the primary body at least partially overlapping the crotch portion (22) of said main body,
and **in that** the sanitary strip (3) is secured to the main panty body (2) by at least two so-called longitudinal seams (4) extending along the longitudinal edges (31) of said strip, each seam (4) being spaced away from the longitudinal edge (31) of the strip closest to said seam to delimit, between said seam and said longitudinal edge, one of said sealing wings (33) so that each of said sealing wings (33) is formed in a single piece with said sanitary strip (3),
and **in that** each of said sealing wings (33) formed in a single piece with the sanitary strip (3) is equipped, over at least part of its length, with elastic (32) for tensioning the free longitudinal edge of the wing, which makes it possible, by shortening the length of said free longitudinal edge (31) of the wing, to lift said sealing wing relative to the portion of the sanitary strip that extends between said wings (33).

2. The garment (1) according to claim 1, **characterized in that** each elastic extends over only part of the length of said wing, preferably over the length of the crotch portion, while being spaced away from the ends of said wing.

3. The garment (1) according to one of the preceding claims, **characterized in that** the ends of the longitudinal edge (31) delimiting each wing are secured, preferably by sewing, to the main panty body, preferably respectively at, or near, the waist opening edges (231, 211) of the front portion (23) and the back portion (21) of the main body.

4. The garment (1) according to one of the preceding claims, **characterized in that** the main panty body is a multi-layer formed from at least one impermeable outer layer and an inner layer.

5. The garment (1) according to the preceding claim, **characterized in that** the inner layer of the main panty body (2) is made from a permeable material, such as cotton fiber.

6. The garment (1) according to one of the preceding claims, **characterized in that** at least the portions of the sanitary strip (3) serving to form the wings are impermeable, on the inner and/or outer side.

7. The garment (1) according to one of the preceding claims, **characterized in that** the sanitary strip comprises, between the two seams (4) securing the main panty body (2), fastening means (5) for attaching an absorbent element equipped with additional fastening means for removable fastening.

8. The garment (1) according to one of the preceding claims, **characterized in that** said sanitary strip comprises an absorption element situated between the two longitudinal seams.

## Patentansprüche

1. Bekleidungsartikel (1) aus der Familie der Hosen, wie Windel, Windelhose, Überhose, Badehose oder Trikotwindel, Slip, wobei der Artikel einen Haupthosenkörper (2) aufweist, der einen vorderen Abschnitt (23) und einen hinteren Abschnitt (21) des Hosenkörpers umfasst, die miteinander durch einen Schrittabschnitt (22) verbunden sind und miteinander verbunden oder verbindbar sind, um im verbundenen Zustand eine Taillenöffnung (13) und zwei Beinöffnungen (11) zu bilden, wobei der Schrittabschnitt mit mindestens einem Flügel ausgestattet ist, der Dichtungsflügel (33) genannt wird, der auf der Seite jeder Beinöffnung eine Abdichtbarriere bildet,
**dadurch gekennzeichnet, dass** der Artikel einen Reinheitsband aufweist, das auf der Innenseite des Haupthosenkörpers angeordnet ist, wobei sich dieses Band (3) längs zwischen den Taillenöffnungsrändern (231, 211) des vorderen Abschnitts (23) und des hinteren Abschnitts (21) des Hauptkörpers erstreckt, indem es mindestens teilweise den Schrittabschnitt (22) des Hauptkörpers bedeckt,
und dass das Reinheitsband (3) mit dem Haupthosenkörper (2) anhand von mindestens zwei Längsnahtlinien (4) verbunden ist, die sich entlang der Längsränder (31) des Bandes erstrecken, wobei jede Nahtlinie (4) vom Längsrand (31) des Bandes, der der Nahtlinie am nächsten ist, beabstandet ist, um zwischen der Nahtlinie und dem Längsrand einen der Dichtungsflügel (33) derart zu definieren, dass jeder der Dichtungsflügel (33) mit dem Reinheitsband (3) von einem einzigen Teil gebildet wird,
und dass jeder der Dichtungsflügel (33), der mit dem Reinheitsband (3) von einem Teil gebildet wird, über mindestens einen Abschnitt seiner Länge mit einem Gummizugband (32) des freien Längsrand des Flügels ausgestattet ist, das erlaubt, indem es die Länge des freien Längsrands (31) des Flügels verkürzt, den Dichtungsflügel im Verhältnis zum dem Abschnitt des Reinheitsbands anzuheben, der sich zwischen den Flügeln (33) erstreckt.

2. Bekleidungsartikel (1) nach Anspruch 1, **dadurch gekennzeichnet, dass** sich jeder Gummi über einen Abschnitt nur der Länge des Flügels erstreckt, vorzugsweise über die Länge des Schrittabschnitts, wobei die Beabstandung durch die Enden des Flügels erfolgt.

3. Bekleidungsartikel (1) nach einem der vorangehenden Ansprüche, **dadurch gekennzeichnet, dass** die Enden des Längsrands (31), der jeden Flügel begrenzt, vorzugsweise durch Nähen mit dem Haupthosenkörper verbunden sind, vorzugsweise an den oder in der Nähe der Taillenöffnungsränder (231, 211) des vorderen Abschnitts (23) und des hinteren Abschnitts (21) des Haupthosenkörpers.

4. Bekleidungsartikel (1) nach einem der vorangehenden Ansprüche, **dadurch gekennzeichnet, dass** der Haupthosenkörper ein Vielschichtkonstrukt ist, das von mindestens einer undurchlässigen Außenschicht und einer inneren Schicht gebildet wird.

5. Bekleidungsartikel (1) nach vorangehendem Anspruch, **dadurch gekennzeichnet, dass** die innere Schicht des Haupthosenkörpers (2) aus durchlässigem Material ist, wie Baumwollfaser.

6. Bekleidungsartikel (1) nach einem der vorangehenden Ansprüche, **dadurch gekennzeichnet, dass** mindestens die Abschnitte des Reinheitsbands (3), die zur Ausbildung der Flügel dienen, innenseitig und/oder außenseitig undurchlässig sind.

7. Bekleidungsartikel (1) nach einem der vorangehenden Ansprüche, **dadurch gekennzeichnet, dass** das Reinheitsband zwischen zwei Befestigungsnähten (4) am Haupthosenkörper (2) Befestigungsmittel (5) eines absorbierenden Elements umfasst, das mit zusätzlichen Befestigungsmitteln für eine lösbare Befestigung ausgestattet ist.

8. Bekleidungsartikel (1) nach einem der vorangehenden Ansprüche, **dadurch gekennzeichnet, dass** das Reinheitsband ein Absorptionselement umfasst, das sich zwischen den zwei Längsnahtlinien befindet.
